(19) Europäisches Patentamt
European Patent Office
Office européen des brevets

(11) EP 0 643 052 B1

(12) **EUROPÄISCHE PATENTSCHRIFT**

(45) Veröffentlichungstag und Bekanntmachung des Hinweises auf die Patenterteilung:
**18.12.1996 Patentblatt 1996/51**

(51) Int. Cl.$^6$: **C07D 309/32**

(21) Anmeldenummer: **94113871.1**

(22) Anmeldetag: **05.09.1994**

# (54) Verfahren zur enantioselektiven Hydrierung von 2H-Pyran-2-on-Derivaten

Process for enantioselective hydrogenation of 2H-Pyran-2-one derivatives

Procédé pour l'hydrogènation énantionsélective des dérivés de 2H-Pyran-2-one

(84) Benannte Vertragsstaaten:
**AT BE CH DE DK ES FR GB IT LI NL**

(30) Priorität: **13.09.1993 CH 2738/93**

(43) Veröffentlichungstag der Anmeldung:
**15.03.1995 Patentblatt 1995/11**

(73) Patentinhaber: **F. HOFFMANN-LA ROCHE AG
4002 Basel (CH)**

(72) Erfinder:
- **Broger, Emil Albin
CH-4312 Magden (CH)**
- **Karpf, Martin
CH-4153 Reinach (CH)**
- **Zutter, Ulrich
CH-4051 Bsel (CH)**

(74) Vertreter: **Kellenberger, Marcus, Dr. et al
F.Hoffmann-La Roche AG
Patent Department (PLP),
124 Grenzacherstrasse
4070 Basel (CH)**

(56) Entgegenhaltungen:
**EP-A- 0 398 132**
**EP-A- 0 443 449**
**EP-A- 0 524 495**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.10/3.4

## Beschreibung

Die vorliegende Erfindung betrifft ein neues, katalytisches Verfahren zur Herstellung von optisch aktiven Verbindungen der allgemeinen Formel

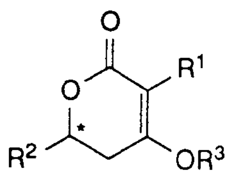

I

worin

| | |
|---|---|
| $R^1$ und $R^2$ | in einer anderen als der α- oder β-Stellung gegebenenfalls durch ein O-Atom unterbrochenes Alkyl, oder gegebenenfalls substituiertes Benzyl bedeutet, |
| $R^3$ | Wasserstoff, niederes Alkyl, gegebenenfalls substituiertes Benzyl, -CO—$R^4$, -$COOR^4$ oder -$CONR^4_2$ bedeutet, und |
| $R^4$ | ein niederes Alkyl oder Aryl darstellt. |

Die Verbindungen der Formel I sind an sich bekannte Verbindungen und wertvolle Zwischenprodukte für pharmakologisch verwendbare Endprodukte. Sie können beispielsweise, wie in EP 443 449 beschrieben, in Pharmazeutika für die Hemmung der Pankreaslipase übergeführt werden.

**EP-A-524 495 beschreibt ein Verfahren zur Herstellung von Verbindungen der Formel I aus chiralen Vorstufen.** Die Aufgabe der vorliegenden Erfindung besteht darin, einen direkten Zugang zu optisch aktiven Verbindungen der Formel I zu finden, durch welchen sich eine nachfolgende Racematspaltung erübrigt.

Das erfindungsgemässe Verfahren ist dadurch gekennzeichnet, dass man eine Verbindung der allgemeinen Formel

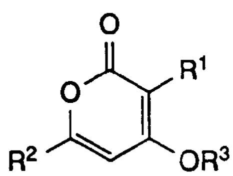

II

worin $R^1$, $R^2$, $R^3$ die obige Bedeutung haben,
in Gegenwart eines Komplexes eines optisch aktiven, vorzugsweise atropisomeren Diphosphin-Liganden mit einem Metall der Gruppe VIII asymmetrisch hydriert.

Als optisch aktive Metall-Diphosphin-Komplexe für das erfindungsgemässe Verfahren kommen insbesondere in Frage optisch aktive Ruthenium Komplexe der allgemeinen Formeln

$$[RuL]^{2+}(X)_2 \quad \text{III-a}$$

$$[RuLX^2]^{2+}(X)_2 \quad \text{III-b}$$

$$[RuLX^1X^2]^{+}X^3 \quad \text{III-c}$$

$$RuL(X^4)_2 \quad \text{III-d}$$

worin

| | |
|---|---|
| X | $BF_{4-}$, $ClO_{4-}$, $B(Phenyl)_{4-}$, $SbF_{6-}$, $PF_{6-}$, $Z^1$-$SO_{3-}$, |
| $X^1$ | Halogenid, |
| $X^2$ | Benzol, Hexamethylbenzol oder p-Cymol, |
| $X^3$ | Halogenid, $ClO_{4-}$, $B(Phenyl)_{4-}$, $SbF_{6-}$, $PF_{6-}$, $Z^1$-$SO_{3-}$ oder $BF_{4-}$, |

| | |
|---|---|
| $X^4$ | ein Anion $Z^2$-$COO^-$ oder ein Anion $Z^3$-$SO_{3^-}$, |
| $Z^1$ | halogeniertes niederes Alkyl oder halogeniertes Phenyl, |
| $Z^2$ | niederes Alkyl, Phenyl, halogeniertes niederes Alkyl oder halogeniertes Phenyl, |
| $Z^3$ | niederes Alkyl oder Phenyl bedeuten, und |
| L | einen optisch aktiven, atropisomeren Diphosphin-Liganden der Formel |

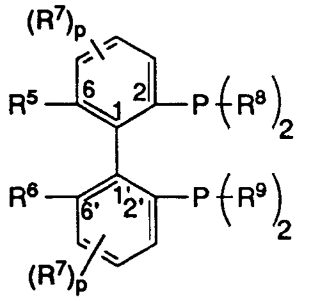

IV

oder der allgemeinen Formel

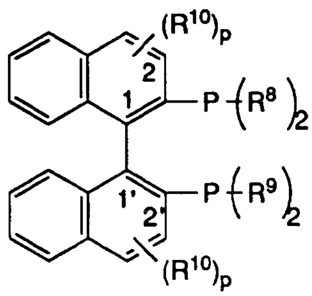

V

in der (S) oder (R) Form darstellt,
worin

| | |
|---|---|
| $R^5$ und $R^6$ | unabhängig voneinander niederes Alkyl, niederes Alkoxy, Di-(niederes Alkyl)-amino, Hydroxy, geschütztes Hydroxy, Hydroxymethyl oder geschütztes Hydroxymethyl, oder |
| $R^5$ und $R^6$ | zusammen eine zweiwertige Gruppe |
| | $-(CH_2)_q-$, $-CH_2-O-CH_2-$, |

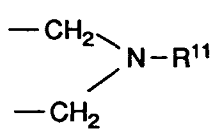

oder

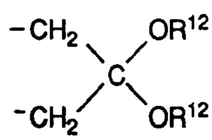

bedeuten,

| | |
|---|---|
| $R^7$ | Wasserstoff, niederes Alkyl oder niederes Alkoxy bedeutet, |
| $R^8$ und $R^9$ | unabhängig voneinander cyclo-Alkyl, unsubstituiertes oder substituiertes Phenyl oder einen fünfgliedrigen Heteroaromaten darstellen, mit der Massgabe, dass mindestens einer der Reste $R^8$ |

und $R^9$ einen substituierten Phenylring oder einen fünfgliedrigen Heteroaromaten darstellt,

$R^{10}$ bevorzugt in 5,5'-Stellung Halogen, Hydroxy, niederes Alkyl, Amino, Acetamido, Nitro oder Sulfo darstellt,

$R^{11}$ niederes Alkyl, Phenyl oder Benzyl bedeutet,

$R^{12}$ niederes Alkyl oder beide $R^{12}$ zusammen eine Di- oder Trimethylengruppe darstellt,

p Null oder die Zahl 1, 2 oder 3 bedeutet, und

q die Zahl 3, 4 oder 5 ist.

Im Zusammenhang mit den Verbindungen der Formeln I bis V besitzen die nachstehenden Definitionen der allgemeinen Ausdrücke ihre Gültigkeit unabhängig davon, ob die fraglichen Ausdrücke allein oder in Kombination erscheinen.

Der Ausdruck "in einer anderen als der $\alpha$- oder $\beta$-Stellung gegebenenfalls durch ein O-Atom unterbrochenes Alkyl" bedeutet im Rahmen der vorliegenden Anmeldung Alkylgruppen mit 1 bis 17 Kohlenstoffatomen wie beispielsweise Methyl, Ethyl, Propyl, Isopropyl, Butyl, Isobutyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Ethoxypropyl, Ethoxybutyl, Propyloxyethyl und dergleichen.

Der Ausdruck "gegebenenfalls substituiertes Benzyl" umfasst Benzyl oder in 2-, 3-, 4-, 5- und/oder 6-Stellung mit Niederalkyl oder Niederalkoxy substituiertes Benzyl wie beispielsweise 4-Methylbenzyl, 4-Methoxybenzyl, 3-Methylbenzyl und dergleichen.

Niederes Alkyl bedeutet im Rahmen der vorliegenden Erfindung geradkettige oder verzweigte Alkylgruppen mit 1 bis 5 Kohlenstoffatomen, wie beispielsweise Methyl, Ethyl, Propyl, Isopropyl, Butyl, Isobutyl, tert.-Butyl, Pentyl, Isopentyl oder tert.-Pentyl. Der Ausdruck "niederes Alkoxy" bedeutet Gruppen, in denen der Alkylrest die vorhergehende Bedeutung hat.

Aryl bedeutet im Rahmen der vorliegenden Anmeldung einen Phenylrest, welcher sowohl unsubstituiert als auch in ortho-, meta- oder para-Stellung ein- oder mehrfach substituiert sein kann. Als Substituenten kommen insbesondere niedere Alkyl- oder niedere Alkoxygruppen oder Halogen, insbesondere Chlor, in Frage. Besonders bevorzugte Arylgruppen sind Phenyl und p-Tolyl.

Als Halogen bzw. Halogenid wurde Fluor, Chlor, Brom oder Jod eingesetzt, insbesondere aber Chlor, Brom oder Jod.

Der Ausdruck "halogeniertes niederes Alkyl" bedeutet im Rahmen der vorliegenden Anmeldung Alkylgruppen mit 1 bis 4 Kohlenstoffatomen und mit einer variablen Anzahl von Halogenatomen, insbesondere Chlor oder Fluor. Besonders bevorzugte halogenierte niedere Alkylgruppen sind beispielsweise perfluorierte und perchlorierte niedere Alkylgruppen wie beispielsweise Trifluormethyl, Pentafluorethyl und dergleichen.

Der Ausdruck "halogeniertes Phenyl" bedeutet vorzugsweise Perfluorphenyl oder Perfluorbiphenyl.

Der Ausdruck "Di-(niederes Alkyl)-amino" bedeutet im Rahmen der vorliegenden Erfindung beispielsweise Dimethylamino, Diethylamino und dergleichen.

Als Schutzgruppen für die Hydroxygruppen kommen, im Rahmen der vorliegenden Erfindung, insbesondere die üblichen etherbildenden Gruppen, wie z.B. Benzyl, Allyl, Benzyloxymethyl, niederes Alkoxymethyl oder auch 2-Methoxyethoxymethyl in Frage.

Cyclo-Alkyl umfasst im Rahmen der vorliegenden Erfindung cyclische Alkylgruppen mit 5 bis 8 Kohlenstoffatomen, insbesondere jedoch cyclo-Pentyl oder cyclo-Hexyl.

Substituiertes Phenyl bezeichnet im Zusammenhang mit Verbindungen der Formel IV und V, ein mit Elektronen-Donor-Gruppen vorzugsweise in 3 und 5-Stellung substituiertes Phenyl wie beispielsweise Gruppen der Formeln

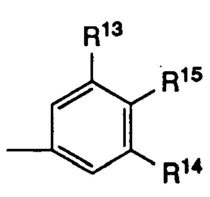

VI-a

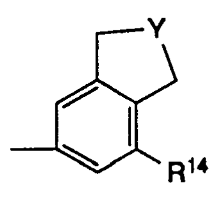

VI-b

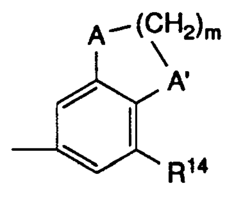

VI-c

worin

Y $(-CH_2-)_m$, Sauerstoff, Schwefel oder $-N-R^{17}$ bedeutet,

A und A' unabhängig von einander $-CH_2-$, $-CR^{13}R^{14}-$, Sauerstoff, Schwefel oder $-N-R^{17}$ bedeuten,

$R^{13}$ und $R^{14}$ unabhängig voneinander niederes Alkyl (wie Methyl, Ethyl, Isopropyl, Isobutyl, tert.-Butyl, Isopentyl

oder tert.-Pentyl), niederes Alkoxy (wie Methoxy, Ethoxy), Trialkylsilyl (wie Trimethylsilyl, Triethylsilyl), cyclo-Alkyl (wie cyclo-Pentyl, cyclo-Hexyl), Benzyl oder einer der Reste $R^{13}$ oder $R^{14}$ auch Wasserstoff bedeuten,

$R^{15}$ Wasserstoff, niederes Alkyl, niederes Alkoxy, Trialkylsilyl (wie beispielsweise Trimethylsilyl oder Triethylsilyl), Di-(niederes Alkyl)-amino oder niederes Thioalkyl darstellt,

$R^{17}$ niederes Alkyl, insbesondere Methyl bedeutet, und

m eine ganze Zahl 1, 2 oder 3 bedeutet.

Der Ausdruck "fünfgliedriger Heteroaromat" steht im Rahmen der vorliegenden Anmeldung, für einen Substituenten der Formel

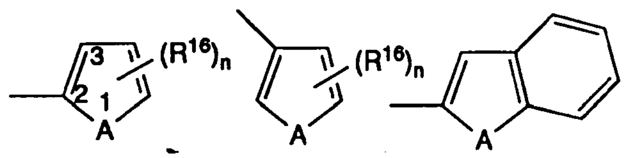
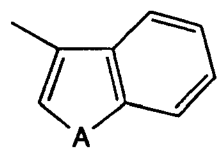

VI-d VI-e VI-f VI-g

In den Formeln VI-d bis VI-g wiederum bedeuten

A Sauerstoff, Schwefel oder -N-$R^{17}$,

$R^{16}$ Wasserstoff, niederes Alkyl, insbesondere Methyl, oder niederes Alkoxy, insbesondere Methoxy,

$R^{17}$ niederes Alkyl, insbesondere Methyl, und

n Null, 1 oder 2.

Die erfindungsgemässe, asymmetrische Hydrierung von Verbindungen der Formel II zu Verbindungen der Formel I kann in einem geeigneten, unter den Reaktionsbedingungen inerten organischen Lösungsmittel erfolgen. Als derartige Lösungsmittel kommen insbesondere in Betracht niedere Alkohole, wie beispielsweise Methanol, Ethanol, isoPropanol; oder Gemische derartiger Alkohole mit halogenierten Kohlenwasserstoffen wie beispielsweise Methylenchlorid, Chloroform, Hexafluorbenzol und dergleichen; oder Gemische mit Ether wie beispielsweise Diethylether, Tetrahydrofuran oder Dioxan, ferner können Gemische obengenanntor Alkohole mit Ester wie beispielsweise Essigsäureethylester, oder mit Carbonsäuren wie beispielsweise Ameisensäure, Essigsäure und dergleichen, oder auch mit Ketonen wie beispielsweise Aceton, Methylethylketon oder Diethylketon, oder mit Wasser verwendet werden.

Vorzugsweise wird die Reaktion in Methanol, Ethanol oder isoPropanol, oder in Gemischen derartiger Alkohole mit obengenannten halogenierten Kohlenwasserstoffen, mit obengenannten Ethern, mit Ketonen oder auch mit Wasser durchgeführt. Insbesondere eignen sich jedoch für das erfindungsgemässe Verfahren obengenannte Alkohole, oder Gemische wie beispielsweise Methanol/ Wasser, oder Methanol/Diethylether/Tetrahydrofuran und dergleichen.

Die Hydrierung wird zweckmässig bei Temperaturen zwischen etwa 0°C und etwa 100°C, vorzugsweise im Temperaturbereich von etwa 20°C bis etwa 80°C,und bei einem Druck von etwa 1 bis etwa 100 bar, vorzugsweise von etwa 5 bis etwa 70 bar, durchgeführt.

Das molare Verhältnis zwischen den zu hydrierenden Verbindungen der Formel II und dem Ruthenium in den Komplexen der Formeln III-a bis III-d liegt zweckmässig zwischen etwa 20.000 und etwa 20, vorzugsweise zwischen etwa 10.000 und etwa 100.

Die Liganden der Formeln IV und V sind bekannte Verbindungen bzw. Analoge bekannter Verbindungen, welche leicht in, zur Herstellung von bekannten Liganden, analoger Weise hergestellt werden können.

Die Verbindungen der Formeln IV und V, worin $R^8$ und $R^9$ gleich sind, können beispielsweise wie in EP 104 375 oder auch in EP 398 132 beschrieben hergestellt werden. Die Herstellung von Verbindungen, worin $R^8$ und $R^9$ voneinander verschieden sind, analog hierzu jedoch in zwei Stufen erfolgt, wie dies beispielsweise in EP 543 245 beschrieben ist.

Die Komplexe der Formeln III-b und III-c können in an sich bekannter Weise hergestellt werden. Komplexe der Formel III-d können z.B. hergestellt werden, indem man Verbindungen der Formeln IV oder V mit Verbindungen, welche Ruthenium abgeben können, in einem geeigneten, inerten organischen oder wässrigen Lösungsmittel umsetzt. Die Komplexe der Formel III-a können aus den Verbindungen III-d durch Umsetzung mit HX, worin X die obengenannte Bedeutung, insbesondere jedoch $BF_{4^-}$, oder $CF_3SO_{3^-}$ bedeutet, hergestellt werden.

Die enantioselektive Hydrierung von Verbindungen der Formel II wird vorzugsweise in Gegenwart von Ruthenium-Diphosphin-Komplexen der Formel III-a, insbesondere in Gegenwart von Ruthenium-Diphosphin-Komplexen der Formel III-a und eines Überschusses an HX oder einer anderen geeigneten Säure wie beispielsweise einer perfluorierten Carbonsäure durchgeführt.

Von den im Rahmen der vorliegenden Erfindung u.a. verwendbaren Liganden der Formeln IV und V, sind diejenigen der Formel IV bevorzugt. Besonders bevorzugte Liganden der Formel IV sind diejenigen, worin $R^5$ und $R^6$ gleich sind und Methyl oder Methoxy bedeuten, p gleich Null ist, und $R^8$ und $R^9$ die gleiche Bedeutung haben und substituiertes Phenyl, vorzugsweise eine Gruppe der Formel VI-a darstellen. Besonders bevorzugt sind Gruppen der Formel VI-a, worin $R^{13}$ und $R^{14}$ gleich sind und Methyl, Isopropyl, tert.-Butyl, tert.-Pentyl, Methoxy, Trimethylsilyl oder Triethylsilyl bedeuten, und $R^{15}$ Wasserstoff, Methoxy oder Dimethylamino bedeutet.

Beispiele besonders bevorzugter Liganden der Formel IV sind

(6,6'-Dimethoxybiphenyl-2,2'-diyl)bis[bis(3,4,5-trimethoxyphenyl)phosphin] (3,4,5-MeO-MeOBIPHEP)
(6,6'-Dimethoxybiphenyl-2,2'-diyl)bis[bis(3,5-dimethyl-4-dimethylaminophenyl)phosphin] (DMAXyl-MeOBIPHEP)
(6,6'-Dimethoxybiphenyl-2,2'-diyl)bis[bis(3,5-bis(trimethylsilyl)phenyl)phosphin] (3,5-TMS-MeOBIPHEP)
(6,6'-Dimethoxybiphenyl-2,2'-diyl)bis[bis(3,5-bis(triethylsilyl)phenyl)phosphin] (3,5-TES-MeOBIPHEP)
(6,6'-Dimethoxybiphenyl-2,2'-diyl)bis[bis(3,5-diisopropylphenyl)phosphin] (3,5-iPr-MeOBIPHEP)
(6,6'-Dimethoxybiphenyl-2,2'-diyl)bis[bis(3,5-ditert.-butylphenyl)phosphin] (3,5-*t*Bu-MeOBIPHEP)
(6,6'-Dimethoxybiphenyl-2,2'-diyl)bis[bis(3,5-ditert.-pentylphenyl)phosphin] (3,5-*t*Pen-MeOBIPHEP)
(6,6'-Dimethoxybiphenyl-2,2'-diyl)bis[bis(3,5-dimethylphenyl)phosphin] (Xyl-MeOBIPHEP)
(6,6'-Dimethoxybiphenyl-2,2'-diyl)bis[bis(3,5-diisopropyl-4-methoxyphenyl)phosphin] (3,5-iPr-4-MeO-MeOBIPHEP)
(6,6'-Dimethoxybiphenyl-2,2'-diyl)bis[bis(3,5-ditert.-butyl-4-methoxyphenyl)phosphin] (3,5-tBu-4-MeO-MeOBIPHEP)
(6,6'-Dimethoxybiphenyl-2,2'-diyl)bis[bis(3,5-ditert.-pentyl-4-methoxyphenyl)phosphin] (3,5-tPen-4-MeO-MeOBIPHEP)

Die folgenden Beispiele dienen zur Erläuterung der Erfindung und stellen keinerlei Einschränkung hiervon dar. In diesen Beispielen haben die verwendeten Abkürzungen folgende Bedeutung:

| | |
|---|---|
| OAc | Acetoxy |
| HPLC | Hochdruck-Flüssigchromatographie |
| RT | Raumtemperatur |
| HV | Hochvakuum: ~ 0,1 mbar |
| GC | Kapillar-Gaschromatographie. Die Proben der Produkte wurden mit Acetanhydrid/Pyridin acetyliert, oder mit N,O-Bis(trimethylsilyl)acetamid/5% Trichlorsilan in Pyridin silyliert. Katalysatorhaltige Proben wurden in Methylenchlorid gelöst und die Lösung durch wenig Kieselgel filtriert, um den Katalysator abzutrennen. |
| o.p. | Optische Reinheit von (R)-3-Hexyl-5,6-dihydro-4-hydroxy-6-undecyl-2H-pyran-2-on, $[\alpha]_D^{20}$ = -45.6° (c=1, Dioxan). |
| ee | Enantiomerer Überschuss von 3-Hexyl-5,6-dihydro-4-methoxy-6-undecyl-2H-pyran-2-on. Der Methylether stammte aus der Hydrierung oder wurde durch Methylierung des entsprechenden Dihydropyrons mittels Orthoameisensäuremethylester/Methanol und p-Toluolsulfonsäure als Katalysator hergestellt. Die ee-Bestimmung erfolgte mittels Hochdruckflüssig-Chromatographie (HPLC) an einer Chiralcel-OD Phase. |
| DHP | 3-Hexyl-5,6-dihydro-4-hydroxy-6-undecyl-2H-pyran-2-on (Dihydropyron) |
| DHPM | 3-Hexyl-5,6-dihydro-4-methoxy-6-undecyl-2H-pyran-2-on (Dihydropyron-Methylether). |

Alle Temperaturen sind in Grad Celsius angegeben.

Beispiel 1

a) In einer Glove Box (Argon, < 1 ppm Sauerstoff) wurden 19.6 mg (0.06 mMol) Di($\eta^2$-acetato)-($\eta^4$-cycloocta-1,5-dien)ruthenium(II) und 61.9 mg (0.060 mMol) (S)-3,5-tBu-MeOBIPHEP in 7.5 ml Diethylether und 2.5 ml Tetrahydrofuran in einem Schlenkrohr gelöst und die Lösung bei 40° über Nacht gerührt. Nach dem Abkühlen wurden 2.5 ml (0.015 mMol) Katalysatorlösung zu 6.5 ml Diethylether/Tetrahydrofuran (3:1, v/v) gegeben und die resultierende Lösung mit einer Lösung von 26.3 mg (0.15 mMol) 50-proz. wässriger $HBF_4$ in 7.5 ml Methanol versetzt. Die gelbe Katalysatorlösung wurde 1.5 h bei Raumtemperatur gerührt.

b) In der Glove Box wurde der Glaseinsatz eines 30-ml-Autoklaven mit 1.05 g (3.00 mMol) 3-Hexyl-4-hydroxy-6-undecyl-2H-pyran-2-on, 13.5 ml Methanol und mit der gemäss a) hergestellten Katalysatorlösung (S/C 200) beladen. Die Hydrierung wurde unter Rühren mit einem Magnetstab während 48 h bei 60° und 60 bar durchgeführt.

Die gelbe Hydrierlösung wurde am Rotationsverdampfer bei 35°/20 mbar eingedampft. Der Rückstand (1.02 g gelbe Kristalle) bestand gemäss GC-Analyse aus 59 % 3-Hexyl-5,6-dihydro-4-hydroxy-6-undecyl-2H-pyran-2-on, 10 % 3-Hexyl-5,6-dihydro-4-methoxy-6-undecyl-2H-pyran-2-on, 5 % eines Gemisches der 4 diastereomeren gesättigten Lactone und 22 % Edukt. Zur Bestimmung der Enantioselektivität wurden 0.71 g des Rückstandes an 40 g Kieselgel chromatographiert. Methylenchlorid eluierte 320 mg (R)-Dihydropyron, $[\alpha]_D^{20}$ = -38.4° und nach Mischfraktionen 27 mg reinen (R)-Dihydropyron-Methylether, 91.9 % ee.

Beispiel 2

a) In einer Glove Box wurde eine Lösung von 15.0 mg (0.012 mMol) $Ru(OAc)_2$[(S)-3,5-tBu-MeOBIPHEP] in 12.5 ml Methanol mit einer Lösung von 21.1 mg (0.12 mMol) 50-proz. wässriger $HBF_4$ in 2.5 ml Methanol versetzt und die Katalysatorlösung 1.5 h bei Raumtemperatur gerührt.

b) In einer Glove Box wurde ein 185-ml-Autoklav mit 4.21 g (12.0 mMol) 3-Hexyl-4-hydroxy-6-undecyl-2H-pyran-2-on, 15 ml Methanol und mit der gemäss a) hergestellten Katalysatorlösung (S/C 1'000) beladen. Die Hydrierung wurde bei 60° und 60 bar und unter kräftigem Rühren während 48 h durchgeführt. Nach dem Abkühlen wurde der gelbe Kristallbrei bei 40°/18 mbar am Rotationsverdampfer eingedampft. Der Rückstand (4.3 g gelbes Kristallisat) der gemäss GC-Analyse aus 73 % 3-Hexyl-5,6-dihydro-4-hydroxy-6-undecyl-2H-pyran-2-on, 20 % 3-Hexyl-5,6-dihydro-4-methoxy-6-undecyl-2H-pyran-2-on und 5 % eines Gemisches der 4 diastereomeren gesättigten Lactone bestand, wurde an 150 g Kieselgel chromatographiert. Methylenchlorid eluierte 210 mg (R)-Dihydropyron, $[\alpha]_D^{20}$ = -42.0° (92 % o.p.), 3.44 g Mischfraktionen bestehend aus Dihydropyron und Dihydropyron-Methylether, und 120 mg (R)-Dihydropyron-Methylether, 92.1 % ee.

## Beispiele 3-10

**Tabelle 1**: Asymmetrische Hydrierungen von 3-Hexyl-4-hydroxy-6-undecyl-2H-pyran-2-on in Gegenwart von $[RuL](BF_4)_2$ als Katalysator in Methanol-Diethylether-Tetrahydrofuran (7 : 2.25 : 0.75, v/v) und bei 60 bar[1)]

| Bsp. Nr. | L | $Ru/HBF_4$ | S/C | T | c | Umsatz | | Selektivität[2)] (DHP + DHPM) | o.p.[3)] | ee[4)] | Konfig. |
|---|---|---|---|---|---|---|---|---|---|---|---|
| | | Mol/Mol | Mol/Mol | °C | % | % | (h) | % | % | % | |
| 3 | (S)-3,5-TMS-MeOBIPHEP | 2 | 25 | 40 | 4 | 37 | (44) | 94 | 91.0 | | R |
| 4 | (S)-DMAXyl-MeOBIPHEP | 2 | 25 | 60 | 4 | 98 | (46) | 95 | 89.5 | 89.5 | R |
| 5 | (S)-3,4,5-MeO-MeOBIPHEP | 10 | 100 | 60 | 4 | 46 | (48) | 78 | | 90.0 | R |
| 6 | (R)-3,5-TES-MeOBIPHEP | 10 | 100 | 60 | 4 | 63 | (51) | 73 | 86.4 | | S |
| 7 | (S)-3,5-tBu-MeOBIPHEP | 10 | 100 | 60 | 4 | 99 | (24) | 74 | | 93.5 | R |
| 8 | (R)-3,5-tBu-MeOBIPHEP | 10 | 1000 | 80 | 15 | 41 | (48) | 90 | | 87.6 | R |
| 9 | (R)-3,5-tBu-4-MeO-MeOBIPHEP | 10 | 1000 | 60 | 15 | 74 | (46) | 95 | | 94.0 | R |
| 10 | (R)-3,5-iPr-4-MeO-MeOBIPHEP | 10 | 1000 | 60 | 15 | 54 | (20) | 98 | - | 93.2 | R |

1) Katalysatorherstellung Bsp. 3 – 7 und 9 und 10 analog Beispiel 1, Bsp. 8 analog Beispiel 2
2) (DHP + DHPM)/Umsatz x 100
3) Optische Reinheit von chromatographisch gereinigtem DHP
4) ee von DHPM

## Beispiele 11-21

**Tabelle 2**: Asymmetrische Hydrierungen von 3-Hexyl-4-hydroxy-6-undecyl-2H-pyran-2-on (c = 15) bei 60 bar[a)]

| Bsp. Nr. | L | HX | Ru/X | S/C | LM v/v | Umsatz % | Umsatz h | Selektivität DHP | Selektivität DHP-Ether | ee % | Konfig. |
|---|---|---|---|---|---|---|---|---|---|---|---|
| 11 | (S)-3,5-tBu-MeOBIPHEP | $BF_4^-$ | 10 | 1000 | $MeOH/Et_2O$ 7:3 | 98 | 43 | 80 | 12 | 93.0 | R |
| 12 | (S)-3,5-tBu-MeOBIPHEP | $BF_4^-$ | 10 | 1000 | $MeOH/H_2O$ 99:1 | 100 | 48 | 88 | 5 | 93.5 | R |
| 13 | (R)-3,5-tBu-MeOBIPHEP | $BF_4^-$ | 10 | 1000 | $MeOH/H_2O$ 97:3 | 99 | 44 | 92 | 1 | 89.6 | S |
| 14 | (R)-3,5-tBu-MeOBIPHEP | $BF_4^-$ | 10 | 1000 | EtOH | 50 | 48 | 80 | 6[b)] | 95.8 | S |
| 15 | (R)-3,5-tBu-MeOBIPHEP | $BF_4^-$ | 10 | 1000 | Isopropanol | 100 | 48 | 97 | <1[c)] | 95.8 | S |
| 16 | (R)-3,5-tBu-MeOBIPHEP | $BF_4^-$ | 10 | 2000 | MeOH | 94 | 48 | 93 | 2 | 91.3 | S |
| 17 | (S)-3,5-tBu-MeOBIPHEP | $BF_4^-$ | 20 | 2000 | MeOH | 58 | 20 | 86 | 4 | 92.4 | R |
| 18 | (S)-3,5-tBu-4-MeO-MeOBIPHEP | $BF_4^-$ | 20 | 2000 | MeOH | 42 | 20 | 87 | 1 | 93.6 | R |
| 19 | (S)-3,5-tBu-4-MeO-MeOBIPHEP | $CF_3SO_3^-$ | 20 | 2000 | MeOH | 29 | 20 | 65 | 20 | 94.2 | R |
| 20 | (S)-3,5-TMS-MeOBIPHEP | $BF_4^-$ | 10 | 1000 | MeOH | 54 | 20 | 87 | 5 | 91.3 | R |
| 21 | (S)-3,5-tBu-4-MeO-MeOBIPHEP | $BF_4^-$ | 20 | 2000 | $MeOH/C_6F_6$ 7:3 | 30 | 20 | 89 | 5 | 94.5 | R |

a) Katalysatorherstellung analog Beispiel 2
b) DHP - Ethylether
c) DHP-isoPropylether

Beispiel 22

a) In einer Glove Box (Argon, < 1 ppm Sauerstoff) wurden 13.1 mg (0.040 mMol) Di($\eta^2$-acetato)-($\eta^4$-cycloocta-1,5-dien)ruthenium(II) und 41.3 mg (0.040 mMol) (S)-3,5-tBu-MeOBIPHEP in 2.25 ml Diethylether und 0.75 ml Tetrahydrofuran in einem Schlenkrohr gelöst und die Lösung bei 40° über Nacht gerührt. Nach dem Abkühlen wurde eine Lösung von 14 mg (0.08 mMol) 50-proz. wässriger $HBF_4$ in 2.5 ml Methanol zugegeben. Die gelbe Katalysatorlösung wurde 1.5 h bei Raumtemperatur gerührt.
b) In der Glove Box wurde der Glaseinsatz eines 30-ml-Autoklaven mit 0.36 g (1.00 mMol) 3-Hexyl-4-methoxy-6-undecyl-2H-pyran-2-on, 4.5 ml Methanol und mit der gemäss a) hergestellten Katalysatorlösung beladen. Die Hydrierung wurde unter Rühren mit einem Magnetstab während 40 h bei 60° und 60 bar durchgeführt.

Die gelbe Hydrierlösung wurde am Rotationsverdampfer bei 35°/20 mbar eingedampft. Der Rückstand (0.35 g gelbe Kristalle) bestand gemäss GC-Analyse aus 71 % 3-Hexyl-5,6-dihydro-4-methoxy-6-undecyl-2H-pyran-2-on und 21 % Edukt. Zur Bestimmung der Enantioselektivität wurde eine Lösung des Rückstandes in Methylenchlorid durch wenig Kieselgel filtriert und das Eluat nach dem Eindampfen im HPLC analysiert: 53 % ee.

Beispiel 23

a) In einer Glove Box wurde eine Lösung von 16.4 mg (0.012 mMol) $Ru(OAc)_2$[(*S*)-3,5-*t*Bu-4-MeO-MeOBIPHEP] in 12.5 ml Methanol mit einer Lösung von 13.7 mg (0.12 mMol) Trifluoressigsäure in 2.5 ml Methanol versetzt und die Katalysatorlösung 1.5 h bei Raumtemperatur gerührt.
b) In einer Glove Box wurde ein 185-ml-Autoklav mit 4.21 g (12.0 mMol) 3-Hexyl-4-hydroxy-6-undecyl-2H-pyran-2-on, 15 ml Methanol und mit der gemäss a) hergestellten Katalysatorlösung (S/C 1'000) beladen. Die Hydrierung wurde bei 60° und 60 bar und unter kräftigem Rühren während 24 h durchgeführt. Nach dem Abkühlen wurde der gelbe Kristallbrei bei 40°/18 mbar am Rotationsverdampfer eingedampft. Der Rückstand (4.2 g gelbes Kristallisat) bestand gemäss GC-Analyse aus 46 % Edukt, 48 % 3-Hexyl-5,6-dihydro-4-hydroxy-6-undecyl-2H-pyran-2-on und 1 % eines Gemisches der 4 diastereomeren gesättigten Lactone.

Zur ee-Bestimmung wurde das Gemisch mit Orthoameisensäuremethylester/Methanol und einer katalytischen Menge p-Toluolsulfonsäure behandelt und im HPLC analysiert: 87.8 % ee (R).

Beispiel 24

a) In einer Glove Box wurde eine Lösung von 16.4 mg (0.012 mMol) $Ru(OAc)_2$[(*S*)-3,5-*t*Bu-4-MeO-MeOBIPHEP] in 12.5 ml Methanol mit einer Lösung von 4.2 mg (0.024 mMol) 50-proz. wässriger $HBF_4$ in 2.5 ml Methanol versetzt und die Lösung 1.5 h bei Raumtemperatur gerührt. Dann wurden 44.6 mg (0.096 mMol) Perfluorpelargonsäure zugegeben und die Katalysatorlösung 15 Min. gerührt.
b) In einer Glove Box wurde ein 185-ml-Autoklav mit 4.21 g (12.0 mMol) 3-Hexyl-4-hydroxy-6-undecyl-2H-pyran-2-on, 15 ml Methanol und mit der gemäss a) hergestellten Katalysatorlösung (S/C 1'000) beladen. Die Hydrierung wurde bei 60° und 60 bar und unter kräftigem Rühren während 20 h durchgeführt. Nach dem Abkühlen wurde der gelbe Kristallbrei bei 40°/18 mbar am Rotationsverdampfer eingedampft. Der Rückstand (4.2 g gelbes Kristallisat) bestand gemäss GC-Analyse aus 51 % Edukt, 44 % 3-Hexyl-5,6-dihydro-4-hydroxy-6-undecyl-2H-pyran-2-on, 2 % 3-Hexyl-5,6-dihydro-4-methoxy-6-undecyl-2H-pyran-2-on und 1 % eines Gemisches der 4 diastereomeren gesättigten Lactone.

Zur ee-Bestimmung wurde das Gemisch mit Orthoameisensäuremethylester/ Methanol und einer katalytischen Menge p-Toluolsulfonsäure behandelt und im HPLC analysiert: 92.8 % ee (R).

**Patentansprüche**

**1.** Verfahren zur enantioselektiven Herstellung von optisch aktiven Verbindungen der Formel

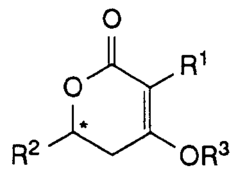

I

worin

$R^1$ und $R^2$ in einer anderen als der α- oder β-Stellung gegebenenfalls durch ein O-Atom unterbrochenes Alkyl **mit 1 bis 17 Kohlenstoffatomen** oder gegebenenfalls substituiertes Benzyl bedeutet,
$R^3$ Wasserstoff, niederes Alkyl mit **1 bis 5 Kohlenstoffatomen,** gegebenenfalls **in 2-, 3-, 4-, 5- und/oder 6 mit $C_1$-$C_5$-Alkyl, $C_1$-$C_5$-Alkoxy** substituiertes Benzyl, -CO—$R^4$, -COOR$^4$ oder -CONR$^4_2$ bedeutet, und
$R^4$ ein niederes Alkyl **mit 1 bis 5 Kohlenstoffatomen** oder **ein gegebenenfalls ein- oder mehrfach mit $C_1$-$C_5$-Alkyl, $C_1$-$C_5$-Alkoxy oder Halogen substituierter Phenylrest** darstellt,

dadurch gekennzeichnet, dass eine Verbindung der allgemeinen Formel

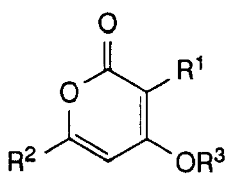

II

worin $R^1$, $R^2$ und $R^3$ die, obengenannten Bedeutungen haben,
in Gegenwart eines Komplexes eines optisch aktiven, atropisomeren Diphosphin-Liganden mit einem Metall der Gruppe VIII asymmetrisch hydriert wird.

**2.** Verfahren gemäss Anspruch 1, dadurch gekennzeichnet, dass die asymmetrische Hydrierung durchgeführt wird in Gegenwart eines optisch aktiven Ruthenium-Diphosphin-Komplexes der Formel

$$[RuL]^{2+}(X)_2 \qquad \text{III-a}$$

$$[RuLX^2]^{2+}(X)_2 \qquad \text{III-b}$$

$$[RuLX^1X^2]^{+}X^3 \qquad \text{III-c}$$

$$RuL(X^4)_2 \qquad \text{III-d}$$

worin

X $BF_{4-}$, $ClO_{4-}$, B(Phenyl)$_{4-}$, $SbF_{6-}$, $PF_{6-}$, $Z^1$-$SO_{3-}$ bedeutet,
$X^1$ Halogenid darstellt,
$X^2$ Benzol, Hexamethylbenzol oder p-Cymol darstellt,
$X^3$ Halogenid, $ClO_{4-}$, B(Phenyl)$_{4-}$, $SbF_{6-}$, $PF_{6-}$, oder $Z^1$-$SO_{3-}$ oder $BF_{4-}$ bedeutet,
$X^4$ ein Anion $Z^2$-$COO^-$ oder ein Anion $Z^3$-$SO_{3-}$ darstellt,
$Z^1$ halogeniertes niederes Alkyl **mit 1 bis 4 Kohlenstoffatomen** oder halogeniertes Phenyl bedeutet,
$Z^2$ niederes Alkyl **mit 1 bis 5 Kohlenstoffatomen,** Phenyl, halogeniertes niederes Alkyl **mit 1 bis 4 Kohlenstoffatomen** oder halogeniertes Phenyl bedeutet,
$Z^3$ niederes Alkyl **mit 1 bis 5 Kohlenstoffatomen** oder Phenyl bedeutet, und
L einen optisch aktiven, atropisomeren Diphosphin-Liganden der Formel

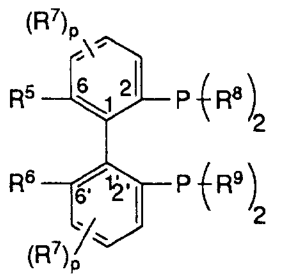

IV

oder der allgemeinen Formel

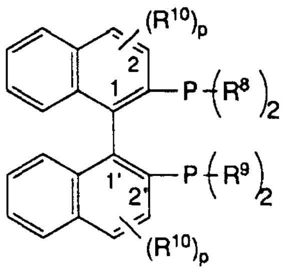

V

in der (R) oder (S) Form darstellt,
worin

| | |
|---|---|
| $R^5$ und $R^6$ | unabhängig voneinander niederes Alkyl, niederes Alkoxy **mit jeweils 1 bis 5 Kohlenstoffatomen,** Di- ($C_1$-$C_5$-Alkyl)-amino, Hydroxy, geschütztes Hydroxy, Hydroxymethyl oder geschütztes Hydroxymethyl oder |
| $R^5$ und $R^6$ | zusammen eine zweiwertige Gruppe $-(CH_2)_q-$, $-CH_2-O-CH_2-$, 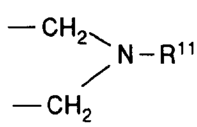 oder 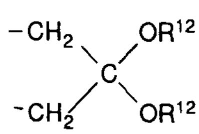 darstellen, |
| $R^7$ | Wasserstoff, niederes Alkyl oder niederes Alkoxy **mit jeweils 1 bis 5 Kohlenstoffatomen** bedeutet, |
| $R^8$ und $R^9$ | unabhängig voneinander cyclo-Alkyl, unsubstituiertes oder substituiertes Phenyl oder einen fünfgliedrigen Heteroaromaten darstellen, mit der Massgabe, dass mindestens einer der Reste $R^8$ und $R^9$ einen substituierten Phenylring oder einen fünfgliedrigen Heteroaromaten darstellt, |
| $R^{10}$ | bevorzugt in 5,5'-Stellung Halogen, Hydroxy, Methyl, Ethyl, Amino, Acetamido, Nitro oder |

Sulfo darstellt,

$R^{11}$ niederes Alkyl **mit 1 bis 5 Kohlenstoffatomen,** Phenyl oder Benzyl bedeutet,

$R^{12}$ niederes Alkyl **mit 1 bis 5 Kohlenstoffatomen** oder beide $R^{12}$ zusammen eine Di- oder Trimethylengruppe darstellt,

p Null oder die Zahl 1, 2 oder 3 bedeutet, und

q für die Zahl 3, 4 oder 5 steht.

**3.** Verfahren gemäss einem der Ansprüche 1 oder 2, dadurch gekennzeichnet, dass die enantioselektive Hydrierung durchgeführt wird in Gegenwart eines optisch aktiven Diphosphin-Komplexes der Formel

$$[RuL]^{2+}(X)_2 \qquad \text{III-a}$$

worin X $BF_{4^-}$ oder $CF_3SO_{3^-}$ bedeutet und L für einen optisch aktiven atropisomeren Liganden der Formel

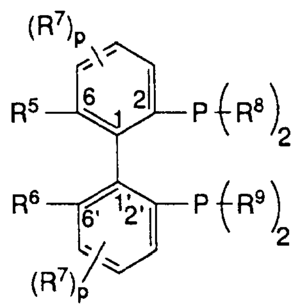

IV

in der (R) oder in der (S) Form steht,
worin

$R^5$ und $R^6$ gleich sind und Methyl oder Methoxy bedeuten, p Null ist,

$R^8$ und $R^9$ gleich sind und ein substituiertes Phenyl darstellen.

**4.** Verfahren gemäss einem der Ansprüche 1 bis 3, dadurch gekennzeichnet, dass $R^8$ und $R^9$ des optisch aktiven Liganden der Formel IV die gleiche Bedeutung haben und einen Rest der Formel

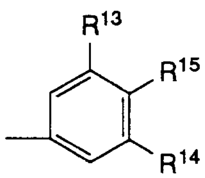

VI-a

darstellen,
worin

$R^{13}$ und $R^{14}$ unabhängig voneinander niederes Alkyl, niederes Alkoxy **mit jeweils 1 bis 5 Kohlenstoffatomen,** Trialkylsilyl, cyclo-Alkyl, Benzyl oder einer der Reste $R^{13}$ oder $R^{14}$ auch Wasserstoff bedeutet, und

$R^{15}$ Wasserstoff, Hydroxy, niederes Alkyl, niederes Alkoxy **mit jeweils 1 bis 5 Kohlenstoffatomen,** Trialkylsilyl oder Di-(niederes **$C_1$-$C_5$**-Alkyl)-amino bedeutet.

**5.** Verfahren gemäss Anspruch 4, dadurch gekennzeichnet, dass $R^{13}$ und $R^{14}$ gleich sind und Methyl, Methoxy, Isopropyl, tert.-Butyl, tert.-Pentyl, oder Trimethylsilyl bedeuten; und $R^{15}$ Wasserstoff oder Methoxy darstellt.

**6.** Verfahren gemäss einem der Ansprüche 1 bis 5, dadurch gekennzeichnet, dass die enantioselektive Hydrierung einer Verbindung der Formel II in einem niederen Alkohol oder in einem Gemisch eines niederen Alkohols mit Ether

oder Wasser bei einer Temperatur zwischen 0° bis 100°C ausgeführt wird.

7. Verfahren nach einem der Ansprüche 1 bis 6, zur Herstellung von Verbindungen der Formel I, worin $R^1$ eine geradkettige Alkylgruppe mit 5-17 Kohlenstoffatomen, $R^2$ eine geradkettige Alkylgruppe mit 1 bis 7 Kohlenstoffatomen und $R^3$ Wasserstoff darstellt, durch enantioselektive Hydrierung einer Verbindung der Formel II in Gegenwart des optisch aktiven Ruthenium-Diphosphin-Komplexes der Formel III-a.

**Claims**

1. A process for the enantioselective manufacture of optically active compounds of the formula

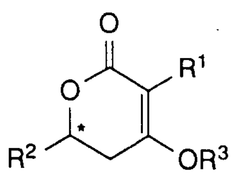

I

wherein

| | |
|---|---|
| $R^1$ and $R^2$ | signify alkyl with 1 to 17 carbon atoms, which is optionally interrupted by an O atom in a position other than the α- or β-position, or optionally substituted benzyl, |
| $R^3$ | signifies hydrogen, lower alkyl with 1 to 5 carbon atoms, benzyl optionally substituted in the 2-, 3-, 4-, 5- and/or 6-position with $C_1$-$C_5$-alkyl, $C_1$-$C_5$-alkoxy, -CO-$R^4$, -COO$R^4$ or -CON$R^4_2$, and |
| $R^4$ | represents a lower alkyl with 1 to 5 carbon atoms or a phenyl residue optionally mono- or multiply-substituted with $C_1$-$C_5$-alkyl, $C_1$-$C_5$-alkoxy or halogen, |

characterized by asymmetrically hydrogenating a compound of the general formula

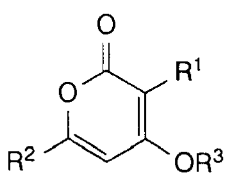

II

wherein $R^1$, $R^2$ and $R^3$ have the significances given above, in the presence of a complex of an optically active, atropisomeric diphosphine ligand with a metal of Group VIII.

2. A process according to claim 1, characterized in that the asymmetric hydrogenation is carried out in the presence of an optically active ruthenium-diphosphine complex of the formula

$$[RuL]^{2+}(X)_2 \qquad \text{III-a}$$

$$[RuLX^2]^{2+}(X)_2 \qquad \text{III-b}$$

$$[RuLX^1X^2]^{+}X^3 \qquad \text{III-c}$$

$$RuL(X^4)2 \qquad \text{III-d}$$

wherein

| | |
|---|---|
| X | signifies $BF_{4-}$, $ClO_{4-}$, B(phenyl)$_{4-}$, $SbF_{6-}$, $PF_{6-}$ or $Z^1$-$SO_{3-}$, |
| $X^1$ | signifies halide, |
| $X^2$ | signifies benzene, hexamethylbenzene or p-cymene, |

$X^3$ signifies halide, $ClO_{4-}$, $B(phenyl)_{4-}$, $SbF_{6-}$, $PF_{6-}$, or $Z^1$-$SO_{3-}$ or $BF_{4-}$,
$X^4$ signifies an anion $Z^2$-$COO^-$ or an anion $Z^3$-$SO_{3-}$,
$Z^1$ signifies halogenated lower alkyl with 1 to 4 carbon atoms or halogenated phenyl,
$Z^2$ signifies lower alkyl with 1 to 5 carbon atoms, phenyl, halogenated lower alkyl with 1 to 4 carbon atoms or halogenated phenyl,
$Z^3$ signifies lower alkyl with 1 to 5 carbon atoms or phenyl and
L signifies an optically active, atropisomeric diphosphine ligand of the formula

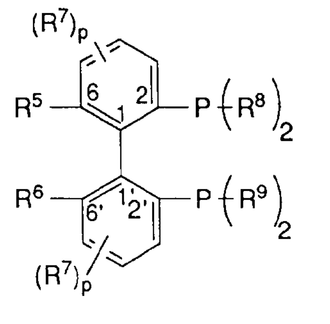

IV

or of the general formula

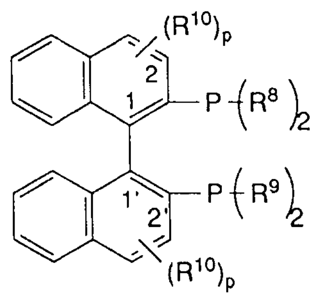

V

in the (S) or (R) form,
wherein

$R^5$ and $R^6$ each independently signify lower alkyl, lower alkoxy with in each case 1 to 5 carbon atoms, di-($C_1$-$C_5$-alkyl)-amino, hydroxy, protected hydroxy, hydroxymethyl or protected hydroxymethyl, or
$R^5$ and $R^6$ together signify a divalent group
$-(CH_2)_q-$, $-CH_2-O-CH_2-$,

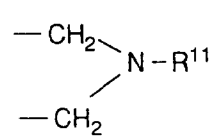

or

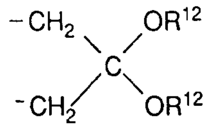

,

| | |
|---|---|
| $R^7$ | signifies hydrogen, lower alkyl or lower alkoxy with in each case 1 to 5 carbon atoms, |
| $R^8$ and $R^9$ | each independently represent cyclo-alkyl, unsubstituted or substituted phenyl or a 5-membered heteroaromatic ring, with the proviso that at least one of the residues $R^8$ and $R^9$ represents a substituted phenyl ring or a 5-membered heteroaromatic ring, |
| $R^{10}$ | represents halogen, hydroxy, methyl, ethyl, amino, acetamido, nitro or sulpho, preferably in the 5,5'-position, |
| $R^{11}$ | signifies lower alkyl with 1 to 5 carbon atoms, phenyl or benzyl, |
| $R^{12}$ | represents lower alkyl with 1 to 5 carbon atoms, or both $R^{12}$'s together represent a di- or trimethylene group, |
| p | signifies zero or the number 1, 2 or 3 and |
| q | is the number 3, 4 or 5. |

**3.** A process according to claim 1 or 2, characterized in that the enantioselective hydrogenation is carried out in the presence of an optically active, diphosphine complex of the formula

$$[RuL]^{2+}(X)_2 \qquad \text{III-a}$$

wherein X signifies $BF_{4-}$ or $CF_3SO_{3-}$ and L stands for an optically active, atropisomeric ligand of the formula

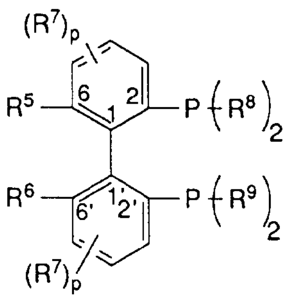

IV

in the (R) or in the (S) form,
wherein

| | |
|---|---|
| $R^5$ and $R^6$ | are the same and signify methyl or methoxy, |
| p | is Zero, and |
| $R^8$ and $R^9$ | are the same and represent a substituted phenyl. |

**4.** A process according to any one of claims 1 to 3, characterized in that $R^8$ and $R^9$ in the optically active ligand of formula IV have the same significance and represent a residue of the formula

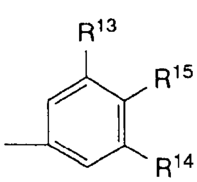

VI-a

wherein

$R^{13}$ and $R^{14}$ each independently signify lower alkyl, lower alkoxy with in each case 1 to 5 carbon atoms, trialkylsilyl, cyclo-alkyl or benzyl or one of the residues $R^{13}$ or $R^{14}$ also signifies hydrogen, and

$R^{15}$ signifies hydrogen, hydroxy, lower alkyl, lower alkoxy with in each case 1 to 5 carbon atoms, trialkylsilyl or di-(lower $C_1$-$C_5$-alkyl)-amino.

**5.** A process according to claim 4, characterized in that $R^{13}$ and $R^{14}$ are the same and signify methyl, methoxy, isopropyl, tert.-butyl, tert.-pentyl or trimethylsilyl; and $R^{15}$ represents hydrogen or methoxy.

**6.** A process according to any one of claims 1 to 5, characterized in that the enantioselective hydrogenation of a compound of formula II is carried out in a lower alcohol or in a mixture of a lower alcohol with ether or water at a temperature between 0° to 100°C.

**7.** A process according to any one of claims 1 to 6 for the manufacture of compounds of formula I in which $R^1$ represents a straight-chain alkyl group with 5-17 carbon atoms, $R^2$ represents a straight-chain alkyl group with 1 to 7 carbon atoms and $R^3$ represents hydrogen by the enantioselective hydrogenation of a compound of formula II in the presence of the optically active ruthenium-diphosphine complex of formula III-a.

## Revendications

**1.** Procédé pour la préparation énantiosélective des composés optiquement actifs de formule

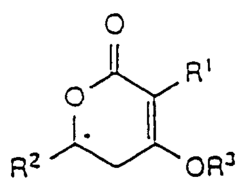

I

où

- $R^1$ et $R^2$ représentent un alkyle comportant 1 à 17 atomes de carbone, éventuellement interrompu par un atome d'O, en une position autre que la position α ou β ou le benzyle éventuellement substitué,
- $R^3$ représente l'hydrogène, un alkyle inférieur comportant 1 à 5 atomes de carbone, le benzyle éventuellement substitué en 2,3,4,5 et/ou 6 par un alkyle en $C_1$-$C_5$ ou par un alcoxy en $C_1$-$C_5$,-CO-$R^4$, -COO$R^4$ ou -CON$(R^4)_2$ et
- $R^4$ représente un alkyle inférieur comportant 1 à 5 atomes de carbone ou un reste phényle éventuellement mono- ou polysubstitué par un alkyle en $C^1$-$C^5$, un alcoxy en $C^1$-$C^5$ ou un halogène,

caractérisé en ce que l'on hydrogène asymétriquement un composé de formule générale

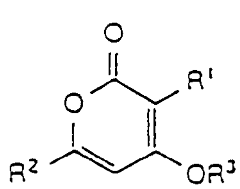

II

où $R^1$, $R^2$ et $R^3$ ont les significations indiquées ci-dessus, en présence d'un complexe d'un ligand diphosphine atropo-isomère optiquement actif, avec un métal du groupe VIII.

**2.** Procédé selon la revendication 1, caractérisé en ce que l'on effectue l'hydrogénation asymétrique en présence d'un complexe diphosphine ruthénium optiquement actif de formule

$$[RuL]^{2+}(X)_2 \qquad \text{III-a}$$

$$[RuLX^2]^{2+}(X)_2 \qquad \text{III-b}$$

$[RuLX^1X^2]^+X^3$ III-c

$RuL(X^4)_2$ III-d

oú

- X représente $BF_{4-}$, $C10_{4-}$, B(phényle)$_{4-}$, $SbF_{6-}$, $PF_{6-}$, $Z^1$ $S0_{3-}$,
- $X^1$ un halogénure,
- $X^2$ le benzène, l'hexaméthylbenzène ou le p-cymène,
- $X^3$ un halogénure, $C10_{4-}$, B(phényle)$_{4-}$, $SbF_{6-}$, $PF_{6-}$ ou $Z^1$-$S0_{3-}$ ou $BF_{4-}$,
- $X^4$ un anion $Z^2$-$C00^-$ ou un anion $Z^3$-$S0_{3-}$,
- $Z^1$ un alkyle inférieur halogéné comportant 1 à 4 atomes de carbone ou un phényle halogéné,
- $Z^2$ un alkyle inférieur comportant 1 à 5 atomes de carbone, le phényle, un alkyle inférieur halogéné comportant 1 à 4 atomes de carbone ou un phényle halogéné,
- $Z^3$ un alkyle inférieur comportant 1 à 5 atomes de carbone ou le phényle et
- L un ligand diphosphine atropo-isomère, optiquement actif, de formule

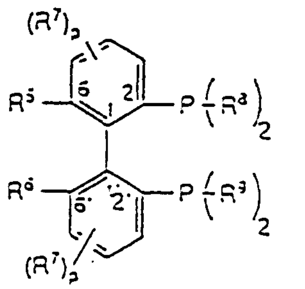

IV

ou de formule générale

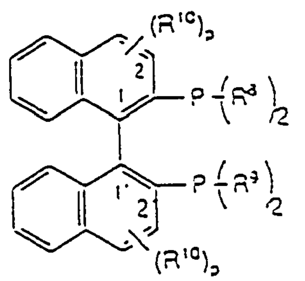

V

sous la forme (R) ou (S).

où

- $R^5$ et $R^6$ représentent indépendamment l'un de l'autre un alkyle inférieur ou un alcoxy inférieur comportant 1 à 5 atomes de carbone, un di (alkyle en $C_1$-$C_5$)amino, un hydroxy, un hydroxy protégé, un hydroxyméthyle ou un hydroxyméthyle protégé

ou

- $R^5$ et $R^6$ représentent ensemble un groupe bivalent $—(CH_2)_q—$. $—CH_2—O\text{-}CH_{2-}$.

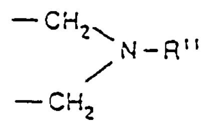

ou

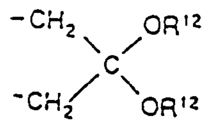

- $R^7$ représente l'hydrogène, un alkyle inférieur ou un alcoxy inférieur comportant 1 à 5 atomes de carbone,
- $R^8$ et $R^9$ représentent, indépendamment l'un de l'autre, un cycloalkyle, un phényle non substitué ou substitué ou un cycle hétéroaromatique à cinq chaînons, sous réserve qu'au moins l'un des restes $R^8$ et $R^9$ représente un noyau phénylique substitué ou un cycle hétéroaromatique à cinq chaînons,
- $R^{10}$ représente un halogène, l'hydroxy, le méthyle, l'éthyle, l'amino, l'acétamido, le nitro ou le sulfo, de préférence, en position 5,5',
- $R^{11}$ un alkyle inférieur comportant 1 à 5 atomes de carbone, le phényle ou le benzyle,
- $R^{12}$ un alkyle inférieur comportant 1 à 5 atomes de carbone ou les deux $R^{12}$ représentent ensemble un groupe di- ou triméthylène,
- p est égal à 0 ou à 1, 2,ou 3 et
- q est égal à 3, 4 ou 5.

3. Procédé selon l'une des revendications 1 et 2, caractérisé en ce que l'on effectue l'hydrogénation énantiosélective en présence d'un complexe diphosphine optiquement actif de formule

$$[RuL]^{2+}(X)_2 \qquad \text{III-a}$$

où X représente $BF^{4-}$ ou $CF_3S0_{3-}$ et L un ligand atropo-isomère, optiquement actif, de formule

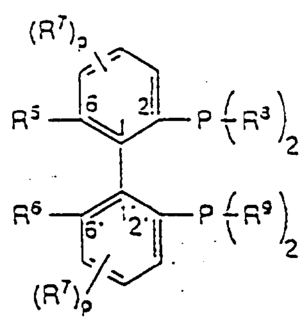

IV

sous la forme (R) ou (S)
où

- $R^5$ et $R^6$ sont identiques et représentent le méthyle ou le méthoxy,
- p est égal à 0,
- $R^8$ et $R^9$ sont identiques et représentent un phényle substitué.

4. Procédé selon l'une des revendications 1 à 3, caractérisé en ce que R8 et R9 du ligand de formule IV optiquement actif ont la même signification et représentent un reste de formule

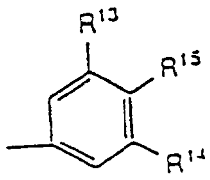

VI a

où

- $R^{13}$ et $R^{14}$ représentent, indépendamment l'un de l'autre, un alkyle inférieur ou un alcoxy inférieur comportant 1 à 5 atomes de carbone, un trialkylsilyle, un cycloalkyle, le benzyle ou l'un des restes $R^{13}$ et $R^{14}$ représente également l'hydrogène et
- $R^{15}$ représente l'hydrogène, l'hydroxy, un alkyle inférieur ou un alcoxy inférieur comportant 1 à 5 atomes de carbone, un trialkylsilyle ou un di(alkyle inférieur en $C_1$-$C_5$) amino.

**5.** Procédé selon la revendication 4, caractérisé en ce que $R^{13}$ et $R^{14}$ sont identiques et représentent le méthyle, le méthoxy, l'isopropyle, le tert-butyle, le tert-pentyle ou le triméthylsilyle et $R^{15}$ représente l'hydrogène ou le méthoxy.

**6.** Procédé selon l'une des revendications 1 à 5, caractérisé en ce que l'on effectue l'hydrogénation énantiosélective d'un composé de formule II dans un alcool inférieur ou dans un mélange d'un alcool inférieur et d'éther ou d'eau à une température comprise entre 0° et 100°C.

**7.** Procédé selon l'une des revendications 1 à 6 pour la préparation des composés de formule I où $R^1$ représente un groupe alkyle à chaîne droite, comportant 5 à 17 atomes de carbone, $R^2$ un groupe alkyle à chaîne droite, comportant 1 à 7 atomes de carbone et $R^3$ l'hydrogène par hydrogénation éniantosélective d'un composé de formule II en présence du complexe diphosphine ruthénium optiquement actif de formule III a.